# EUROPÄISCHE PATENTSCHRIFT

(11) **EP 2 105 734 B1**
(45) Veröffentlichungstag und Bekanntmachung des Hinweises auf die Patenterteilung: **20.11.2013**
(21) Anmeldenummer: 08005615.3
(22) Anmeldetag: 26.03.2008
(51) Int. Cl.: G01N 27/414, G01N 27/00

(54) **Gassensor**
Gas sensor
Capteur de gaz

(43) Veröffentlichungstag der Anmeldung: 30.09.2009
(73) Patentinhaber: Micronas GmbH, 79108 Freiburg i. Br. (DE)
(72) Erfinder: Wilbertz, Christoph, 79194 Gundelfingen (DE); Frerichs, Heinz-Peter, 79271 St. Peter (DE); Senft, Christoph, 80337 München (DE)
(74) Vertreter: Koch, Bertram

(56) Entgegenhaltungen:
- EP-A- 1 879 023
- DE-A1- 4 333 875
- US-A1- 2004 173 004
- US-A1- 2006 196 246
- GALONSKA T ET AL: "Cross Sensitivity and Stability of FET - Based Hydrogen Sensors" SENSORS, 2007 IEEE, IEEE, PI, 28. Oktober 2007 (2007-10-28), Seiten 1036-1039, XP031221242 ISBN: 978-1-4244-1261-7

## Beschreibung

Die Erfindung betrifft einen Gassensor nach dem Oberbegriff von Anspruch 1.

Ein derartiger Gassensor ist aus EP 1 879 023 A1 und T. Galonska et al., "Cross Sensitivity and Stability of FET - Based Hydrogen Sensors", SENSORS, 2007 IEEE, IEE (2007-10-28), 1036-1039 bekannt. Der Gassensor hat eine gassensitive Schicht, die einen Oberflächenbereich aufweist, in dem die Austrittsarbeit von der Konzentration von mit dem Oberflächenbereich in Kontakt bringbarem Zielgas, nämlich Wasserstoff. abhängig ist. Zur Erfassung eines elektrischen Messsignals ist ein Potentialsensor über einen Luftspalt kapazitiv an den Oberflächenbereich gekoppelt. Der Oberflächenbereich ist mit einer für das Wasserstoffgas inerten, elektrisch isolierenden SnO₂-Beschichtung überdeckt, die haftend mit der gassensitiven Schicht verbunden ist. Die Beschichtung ist derart ausgestaltet, dass sie für ein weiteres Gas, nämlich Sauerstoff, durchlässig ist. Dabei weist die Beschichtung für das Zielgas und das weitere Gas unterschiedliche Diffusionskonstanten auf. Unterhalb einer Temperatur von 60° C tritt eine Potentialverschiebung des Messsignals durch dissotiative Adsorption von Wasserstoff auf, die durch eine Abnahme der Sauerstoffbelegung des Oberflächenbereichs verursacht wird. Die adsorbierte Wasserstoff-Schicht ist elektronegativ, wodurch die Austrittsarbeit nichtlinear reduziert wird. Die Veränderung der Austrittsarbeit, die durch die Adsorption von Sauerstoff verursacht wird, ist positiv sowie linear zur Sauerstoffbelegung und geht bei etwa 400 meV in die Sättigung.

Aus US 2006/0196246 A1 ist ferner ein wasserstoffempfindlicher Gassensor bekannt, der eine MIS-Struktur aufweist. Die MIS-Struktur hat eine katalytische Schicht, an der Oberfläche eine wasserstoffselektive Polyimidschicht abgeordnet ist, deren Schicht dicke zwischen 200 und 800 nm beträgt. Nach Angabe der Offenlegungsschrift ermöglicht der Gassensor gegenüber einem unbeschichteten MIS-Gassensor in Gasgemischen eine höhere Messempfindlichkeit für Wasserstoff.

Aus US 2004/0173004 A1 ist ein Gassensor bekannt, der einen Lichtleiter hat, der an seiner Mantelfläche ein Bragg-Gitter aufweist, durch das Licht in den Lichtleiter eingekoppelt wird. Der Lichtleiter ist mit einer Manschette aus für Wasserstoffgas sensitivem Palladium umhüllt, die an den Stellen, an denen sie den Lichtleiter nicht berührt, mit einer wasserstoffundurchlässigen Zeolith-Passivierungsschicht beschichtet ist. Dadurch soll die Gefahr einer sogenannten Vergiftung des Gassensors reduziert werden. Durch die Ausdehnung der Palladium-Manschette wird auf den Lichtleiter und das Bragg-Gitter eine mechanische Spannung ausgeübt, welche die optischen Eigenschaften des Lichtleiters verändert.

Aus DE 43 33 875 C2 ist ferner ein Gassensor zur Messung der Konzentration von Wasserstoffgas bekannt, der ein Silizium-Substrat aufweist, in das ein Feldeffekttransistor integriert ist. Der Feldeffekttransistor weist eine Gate-Elektrode auf, die leitend mit einer Sensorelektrode verbunden ist, über der eine gassensitive Schicht angeordnet ist, die durch einen Luftspalt von der Sensorelektrode beabstandet und über den Luftspalt kapazitiv an die Sensorelektrode gekoppelt ist. Auf die der Sensorelektrode abgewandte Rückseite der gassensitiven Schicht ist eine Deckelektrode aufgebrocht Ein der Sensorelektrode zugewandter Oberflächenbereich der gassensitiven Schicht ist mit Wasserstoffgas in Kontakt bringbar, das beim Kontaktieren das Oberflächenbereichs an diesen adsorbiert. Bei einer Änderung der Konzentration des Wasserstoffgases verändert sich die Austrittsarbeit in dem Oberflächenbereich der gassensitiven Schicht. Da die Sensorelektrode kapazitiv an den Obeflachenbereich gekoppelt ist, verändert sich dabei auch das elektrische Potential an der Gate-Elektrode. In Abhängigkeit von der Potentialänderung wird der Stromfluss zwischen einem Drain- und einem Source-Anschluss des Feldeffekttransistors gesteuert.

In normaler Raumluft ist an der Oberfläche der gassensitiven Schicht eine dünne Schicht Luftsauerstoff dissoziativ adsorbiert, d.h. als Sauerstoffatome, nicht als Sauerstotfmoleküle, wie sie in der Luft vorkommen. Wenn das Wasserstoffgas in die Umgebung der gassensitiven Schicht kommt, findet zunächst eine Adsorption des Wasserstoffgases an der Oberfläche statt, wobei das Wasserstoffgas teilweise den an der Oberfläche adsorbierten Luftsauerstoff verdrängt und dessen Adsorptionsplätze einnimmt. Beide Effekte, Adsorption des Wasserstoffgases und Reduktion des Sauerstoffbelegung tragen additiv zur Änderung der Oberflächen-Austrittsarbeit bei. Gleichzeitig setzt aber an der Oberfläche, gefördert durch die katalytische Wirkung der gassensitiven Schicht eine Reaktion zwischen Wasserstoff und Sauerstoff ein, bei der Wasser gebildet wird. Bei niedrigen Temperaturen unter ca. 60 °C wird dadurch allmählich nur die Wasserstoffbedeckung an der Oberfläche verringert, bei höheren Temperaturen über ca. 60 °C läuft die Reaktion so schnell ab, dass dadurch zusätzlich auch die Wasserstoffkonzentration in der unmittelbaren Umgebung der gassensitiven Schicht verringert wird. Dadurch kann die Sauerstoffbedeckung der Oberfläche wieder zunehmen. Alle drei Effekte verschieben die Austrittsarbeit in entgegengesetzte Richtung. Diese Reaktion kann je nach Temperatur der gassensitiven Schicht über Stunden oder auch in Sekunden stattfinden, so dass das Messsignal stark gestört werden kann. Außerdem nimmt bei dem Gassensor die Sensitivität für das Wasserstoffgas mit zunehmender Wasserstoffgaskonzentration etwa logarithmisch ab. Die Auswertung des Gassensor-Messsignals ist deshalb insbesondere bei höheren Wasserstoffgaskonzentrationen schwierig.

Aufgabe der Erfindung ist es, einen Gassensor der eingangs genannten Art zu schaffen, der es ermöglicht, eine vorbestimmte Wasserstoffgaskonzentration mit großer Präzision zu messen.

Diese Aufgabe wird mit den Merkmalen des Anspruchs 1 gelöst. Unter dem Begriff "Messempfindlichkeit" wird der Betrag der Änderung des Sensorsignals des Potentialsensors dividiert durch den Betrag der Änderung der Gaskonzentration nach Abklingen eventueller, in dem Messsignal enthaltener transienter Signalanteile verstanden.

Bei einem Gassensor mit der erfindungsgemäßen Beschichtung kann beim Überschreiten des Konzentrations-Schwellenwerts ein nahezu sprunghafter Anstieg des Sensorssignals des Potenfialsensors erreicht werden. Außerdem hat sich herausgestellt, dass der Gassensor mit dieser Beschichtung auch bei hohen Temperaturen bis ca. 180 °C und/oder in feuchter Umgebung langzeigstabil ist. In entsprechender Weise nimmt die Sensitivität für das Wasserstoffgas stark ab, wenn das Wasserstoffgas bei etwa konstanter Konzentration des Sauerstoffgases den Konzentrations-Schwellenwert unterschreitet. Außerdem werden durch die Beschichtung Störungen im Messsignal des Gassensors, die auf das Wechselspiel von Wasserstoffgas und Sauerstoffgas zurückzuführen sind, weitgehend vermieden. In vorteilhafter Weise ist es dadurch möglich, mit großer Präzision zu detektieren, ob die Wasserstoffgaskonzentration über oder unter dem Konzentrations-Schwellenwert liegt. Der Gassensor kann insbesondere als Leckagesensor verwendet werden.

Bei einer bevorzugten Ausführungsform der Erfindung beträgt die Dicke der Beschichtung zwischen 0,3 µm und 4 µm, insbesondere zwischen 0,5 µm und 2,5 µm. Mit dieser Schichtdicke wird eine günstige Messsignalcharakteristik des Gassensors für das Wasserstoffgas ermöglicht.

Bei einer vorteilhaften Ausgestaltung der Erfindung ist die Höhe des Konzentrations-Schwellenwerts von der Temperatur der gassensitiven Schicht und der Beschichtung abhängig und der Gassensor weist zum Einstellen der Temperatur der gassensitiven Schicht und der Beschichtung eine Temperiereinrichtung, vorzugsweise eine Heizung, auf. Durch Beheizen und/oder Kühlen der gassensitiven Schicht kann dann der Konzentrations-Schwellenwert an einen gewünschten Wert angepasst werden.

Bei einer Weiterbildung der Erfindung weist der Gassensor einen Sollwertgeber und eine Regeleinrichtung auf, die einen mit dem Potentialsensor verbundenen Istwerteingang, einen mit einem Sollwertgeber verbundenen Sollwerteingang und einen mit der Temperiereinrichtung verbundenen Stellsignalausgang aufweist, wobei zum Anpassen des Konzentrations-Schwellenwerts an die Wasserstoffgaskonzentration mit Hilfe des Sollwertgebers ein Sollwert an den Sollwerteingang anlegbar ist, der einem Wert entspricht, den das Sensorssignal des Potentialsensors am Konzentrations-Schwellenwert und/oder bei einer Wasserstoffgaskonzentration oberhalb des Konzentrations-Schwellenwerts aufweist. Somit können unterschiedliche Wasserstoffgaskonzentrationen mit großer Präzision gemessen werden, indem der Konzentrations-Schwellenwert jeweils so eingestellt wird, dass er mit der Wasserstoffgaskonzentration übereinstimmt oder etwas kleiner ist als diese.

Bei einer bevorzugten Ausgestaltung der Erfindung besteht die gassensitive Schicht aus Platin oder Palladium. Der Gassensor weist dann für Wasserstoffgas eine hohe Detektionsempfindlichkeit auf.

Der Potentialsensor ist vorzugsweise ein Feldeffekttransistor, der ein Substrat aufweist, auf dem eine Drain und eine Source angeordnet sind, wobei zwischen Drain und Source ein Kanalbereich gebildet ist, und wobei der Kanalbereich direkt über den Luftspalt oder indirekt über eine mit dem Kanalbereich zusammenwirkende Gateelektrode und eine mit der Gateelektrode leitend verbundene Sensorelektrode kapazitiv an den Oberflächenbereich der gassensitiven Schicht gekoppelt ist. Der Feldeffekttransistor kann also ein SGFET oder ein CCFET sein. Der Gassensor ermöglicht dadurch eine kompakte Bauform und lässt sich außerdem gut in einen Halbleiterchip integrieren. Dabei kann in den Halbleiterchip auch eine Auswerteeinrichtung zur Verarbeitung der Messsignale des Gassensors integriert sein.

Der Gassensor kann auch als Kelvinsonde ausgestaltet sein, bei welcher der Potentialsensor über eine durch den Luftspalt von dem Oberflächenbereich der gassensitiven Schicht beabstandete, auf die gassensitive Schicht zu- und von dieser wegbewegbaren Elektrode kapazitiv an den Oberflächenbereich der gassensitiven Schicht gekoppelt ist. Dabei kann die Elektrode beispielsweise mittels eines Piezo-Aktors relativ zu der gassensitiven Schicht positioniert und in Schwingungen versetzt werden. Der Potentialsensor ist einer Auswerte- und Ansteuereinrichtung zugeordnet, die eine Gegenspannung an die Elektrode anlegt, die derart gewählt ist, dass das von dem Potentialsensor gemessene Potential im Mittel gleich null ist. Die Gegenspannung ist ein Maß für die Konzentration des mit dem Oberflächenbereich der gassensitiven Schicht in Kontakt stehenden Wasserstoffgases.

Nachfolgend sind Ausführungsbeispiele der Erfindung anhand der Zeichnung näher erläutert. Es zeigt
- Fig. 1: einen Querschnitt durch einen Gassensor, der einen SGFET aufweist, dessen Kanalbereich über einen Luftspalt kapazitiv an eine mit einer Passivierungs-Beschichtung versehene gassensitive Schicht gekoppelt ist,
- Fig. 2: einen Querschnitt durch einen Gassensor, der einen CCFET aufweist, dessen Sensorelektrode über einen Luftspalt kapazitiv an eine mit einer Passivierungs-Beschichtung versehene gassensitive Schicht gekoppelt ist,
- Fig. 3: einen Querschnitt durch einen als Kelvinsonde ausgestalteten Gassensor, bei dem die gassensitive Schicht eine Passivierungs-Beschichtung aufweist,
- Fig. 4: eine graphische Darstellung eines Sensorssignals (obere Kurve) und der Wasserstoffgaskonzentration (untere Kurve) eines Ausführungsbeispiels des Gassensors, wobei auf der Abszisse die Zeit t und auf der Ordinate links die Amplitude S des Sensorssignals eines Potentialsensors und rechts die Wasserstoffgaskonzentration k aufgetragen sind,
- Fig. 5: eine Darstellung ähnlich Fig. 4, wobei jedoch die Temperatur des Gassensors geringer ist als in Fig. 4,
- Fig.6: eine Darstellung ähnlich Fig. 5, wobei jedoch die Temperatur des Gassensors geringer ist als in Fig. 5,
- Fig. 7: eine graphische Darstellung eines Schwellenwerts für die Wasserstoffgaskonzentration des Gassensors, wobei auf der Abszisse die Temperatur und auf der Ordinate der Schwellenwert aufgetragen sind, und
- Fig. 8: eine schematische Darstellung einer Regeleinrichtung.

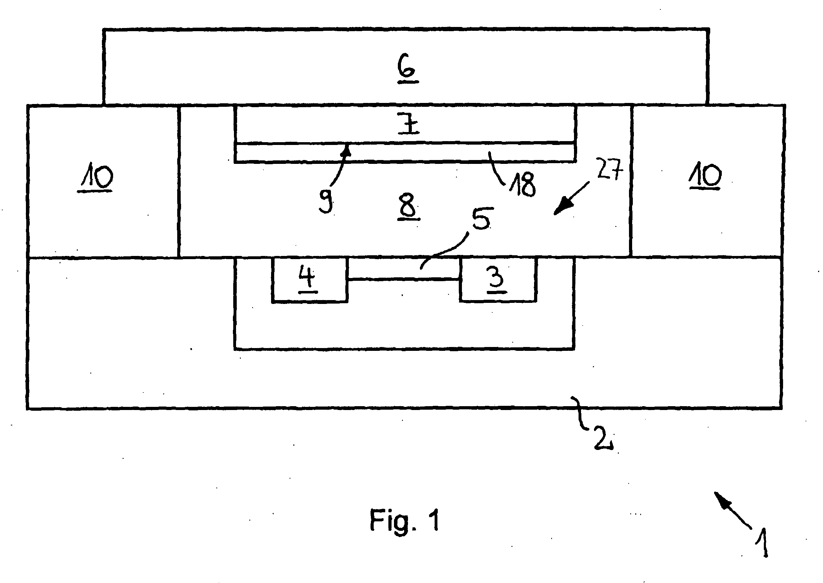

Ein in Fig. 1 im Ganzen mit 1 bezeichneter Gassensor weist ein Substrat 2 auf, in das ein Potentialsensor 27 integriert ist. Der Potentialsensor 27 hat eine Drain 3 und eine Source 4, die in einer n-dotierten Transistorwanne angeordnet sind. Die Drain 3 und die Source 4 können beispielsweise aus p-dotiertem Silizium bestehen. Die Drain 3 ist über elektrische Leiterbahnen mit einem in der Zeichnung nicht näher dargestellten Drain-Anschluss verbunden. In entsprechender Weise ist die Source 4 mit einem Source-Anschluss verbunden. Zwischen Drain 3 und Source 4 ist in dem Substrat 2 ein Kanalbereich 5 gebildet, auf dem eine elektrisch isolierende Dünnoxidschicht angeordnet ist, die als Gatedielektrikum dient.

Über dem Kanalbereich 5 ist an einem Trägerteil 6 eine gassensitive Schicht 7 angeordnet, die beispielsweise aus einem Edelmetall, insbesondere aus Platin oder Palladium besteht und durch einen Luftspalt 8 von dem Kanalbereich 5 beabstandet ist. Ein dem Kanalbereich 5 zugewandter Oberflächenbereich 9 der gassensitiven Schicht 7 ist über den Luftspalt 8 kapazitiv an den Kanalbereich 5 gekoppelt.

Das Trägerteil 6 ist beidseits der gassensitiven Schicht 7 über eine elektrische Isolationsschicht 10 mit dem Substrat 2 verbunden. In Fig. 1 ist deutlich erkennbar, dass das Trägerteil 6 und die gassensitive Schicht 7 ein Suspended Gate bilden.

Der Luftspalt 4 ist über mindestens eine in der Zeichnung nicht näher dargestellte Öffnung mit der den Gassensor 1 umgebenden Atmosphäre verbunden. Über diese Öffnung kann der Oberflächenbereich 9 der gassensitiven Schicht 7 mit zu detektierenden Wasserstoffgas, und mit z.B. in der Atmosphäre enthaltendem Sauerstoffgas, in Kontakt gebracht werden. Bei einem Kontakt mit dem Oberflächenbereich 9 adsorbieren das Wasserstoffgas und das Sauerstoffgas an dem Oberflächenbereich 9. Bei der Adsorption des Wasserstoffgases verändert sich in dem Oberflächenbereich 9 die Austrittsarbeit, was zu einer Veränderung des elektrischen Potentials in dem Kanalbereich 5 führt.

Bei dem Ausführungsbeispiel nach Fig. 1 ist der Kanalbereich 5 offen ausgebildet (ISFET) und über das Dünnschichtoxid und den Luftspalt 8 direkt an die gassensitive Schicht 7 kapazitiv gekoppelt. Deutlich ist erkennbar, dass der Kanalbereich 5 an der der gassensitiven Schicht 7 gegenüberliegenden Seite des Luftspalts 8 angeordnet ist.

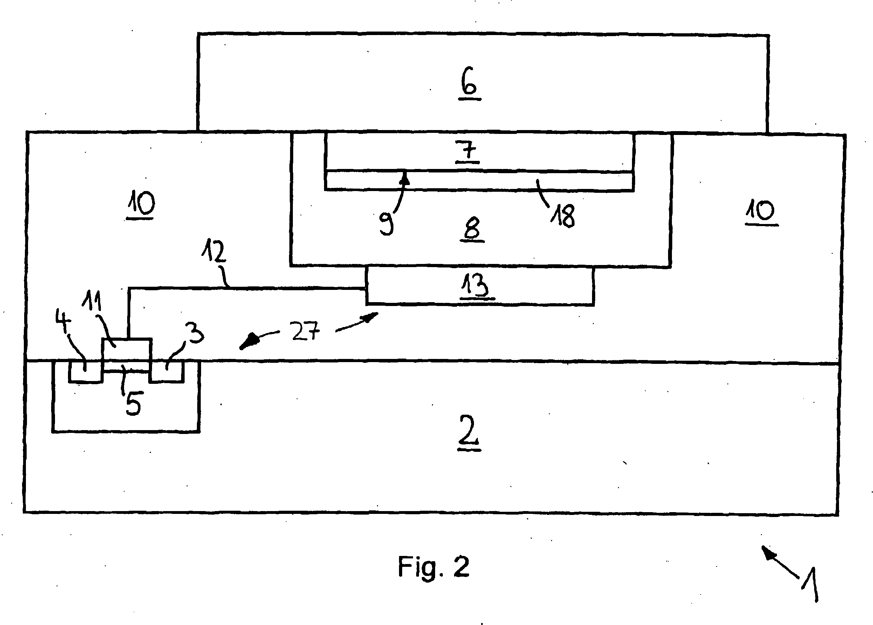

Bei dem Ausführungsbeispiel nach Fig. 2 ist der Feldeffekttransistor als CCFET ausgestaltet, bei dem der Kanalbereich 5 seitlich neben der gassensitiven Schicht 7 in dem Substrat 2 angeordnet und mit einer Gateelektrode 11 abgedeckt ist. Zur kapazitiven Ankopplung des Kanalbereichs 5 an die gassensitive Schicht 7 ist die Gateelektrode 11 über eine elektrische Verbindungsleitung 12 mit einer Sensorelektrode 13 verbunden, die an der dem Oberflächenbereich 9 der gassensitiven Schicht 7 gegenüberliegenden Seite des Luftspalts 8 auf einer auf dem Substrat 2 befindlichen Isolationsschicht 10 angeordnet ist. Die Isolationsschicht 10 kann beispielsweise eine SiO₂-Schicht sein. Der Aufbau des Suspended Gates des SGFET entspricht dem in Fig. 1.

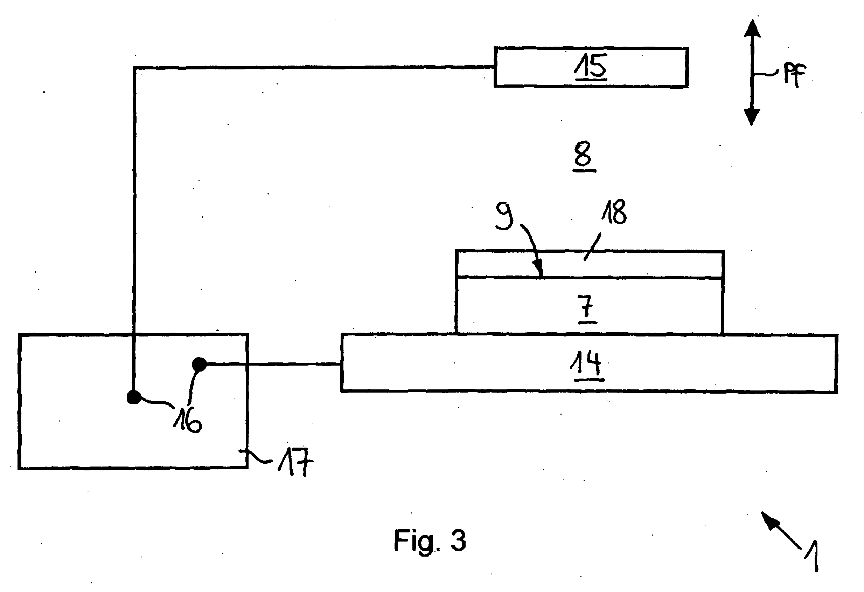

Bei dem in Fig. 3 gezeigten Ausführungsbeispiel ist der Gassensor 1 als Kelvin-Sonde ausgestaltet. Die gassensitive Schicht 7 ist auf einem elektrisch leitenden Träger 14 angeordnet und weist an ihrer dem Träger 14 abgewandten Seite einen Oberflächenbereich 9 auf, an den das Wasserstoffgas adsorbieren kann. Der Oberflächenbereich 9 ist durch einen Luftspalt 8 von einer Elektrode 15 beabstandet und bildet mit dieser eine elektrische Kapazität.

Die Elektrode 15 kann mit Hilfe eines in der Zeichnung nicht näher dargestellten Aktors in Schwingungen versetzt werden. Dabei bewegt sich die Elektrode 15 entsprechend dem Pfeil Pf abwechselnd auf die gassensitive Schicht 7 zu- und von dieser weg. Die Elektrode 15 und der Träger 14 bzw. die gassensitive Schicht 7 sind mit Anschlüssen 16 einer Auswerte- und Ansteuereinrichtung 17 verbunden. Diese hat einen in Fig. 3 nicht näher dargestellten Potentialsensor, der zur Messung der elektrischen Spannung zwischen der gassensitiven Schicht 7 und der Elektrode 15 mit den Anschlüssen 16 verbunden ist. Die Auswerte- und Ansteuereinrichtung 17 weist außerdem eine mit dem Potentialsensor in Steuerverbindung stehende, verstellbare Spannungsquelle auf, mittels der eine Gegenspannung zwischen den Potentialsensor und die Elektrode 15 und/oder den Träger 15 anlegt wird. Die Gegenspannung wird so gewählt, dass das von dem Potentialsensor gemessene Potential im Mittel gleich null ist.

Bei den vorstehend beschriebenen Ausführungsbeispielen des Gassensors 1 ist jeweils der Oberflächenbereich 9 der gassensitiven Schichten 7 durchgängig von einer für das Wasserstoffgas inerten, elektrisch isolierenden Beschichtung 18 überdeckt, die aus Polymethylmethacrylat (PMMA) besteht. Die Beschichtung 18 haftet fest an der gassensitiven Schicht 7 an. Die Beschichtung 18 ist als Dickschicht mit einer etwa konstanten Dicke ausgestaltet, die vorzugsweise zwischen 0,5 µm und 2,5 µm beträgt.

Die Beschichtung 18 ist sowohl für das Wasserstoffgas als auch für das Sauerstoffgas durchlässig. Die Beschichtung 18 hat jedoch für das Wasserstoffgas eine andere Diffusionskonstante als für das Sauerstoffgas. Die Diffusionskonstanten, das Wasserstoffgas und das Sauerstoffgas sind derart aufeinander abgestimmt, dass die Sensitivität des Gassensors 1 für das Wasserstoffgas stark zunimmt, wenn die Konzentration des Wasserstoffgases bei Anwesenheit des Sauerstoffgases einen Schwellenwert 19 überschreitet. Die Lage des Schwellenwerts 19 ist von der Temperatur abhängig.

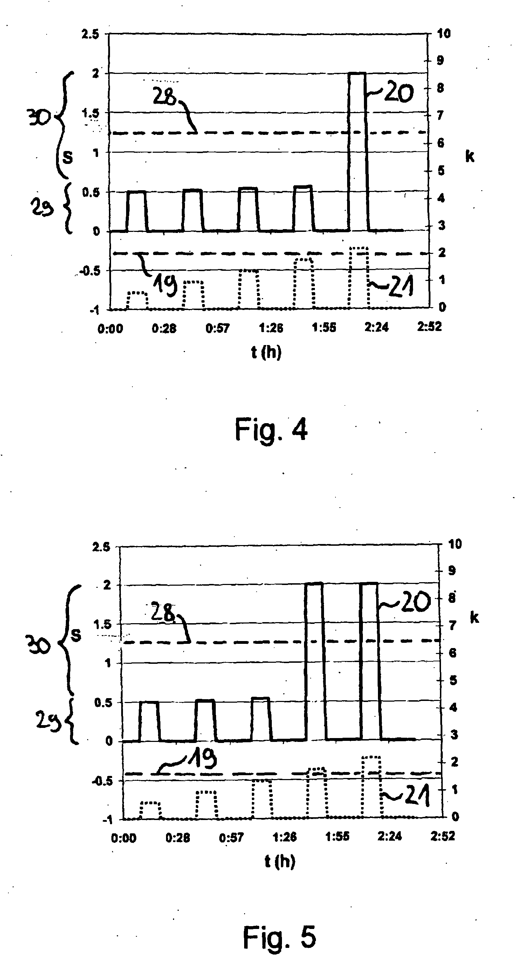

In Fig. 4 bis 6 ist erkennbar, dass das Sensorsignal 20 des Potentialsensors 27 jeweils bei konstanter Temperatur bei Wasserstoffgaskonzentrationen, die in einem ersten Konzentrationsbereich liegen, der nach oben durch den Konzentrations-Schwellenwert 19 begrenzt ist, zunächst etwa logarithmisch mit der Wasserstoffgaskonzentration 21 ansteigt. In dem ersten Konzentrationsbereich liegt das Sensorsignal 20 des Potentialsensors 27 in einem ersten Aussteuerungsbereich 29.

In einem zweiten Konzentrationsbereich, der an seinem unteren Ende an den Konzentrations-Schwellenwert angrenzt und wesentlich schmaler ist als der erste Konzentrationsbereich, steigt das Sensorsignal 20 bei konstanter Temperatur stark an. In dem zweiten Konzentrationsbereich liegt das Sensorsignal 20 in einem zweiten Aussteuerungsbereich 30, in dem die Messempfindlichkeit des Gassensors 1 größer ist als in dem ersten Aussteuerungsbereich 29. In einem dritten Konzentrationsbereich, der oberhalb des zweiten Konzentrationsbereichs liegt und an diesen angrenzt, ist das Sensorsignal 20 des Potentialsensors 27 bei konstanter Temperatur im Wesentlichen konstant, auf einem Wert, der an den zweiten Konzentrationsbereich angrenzt.

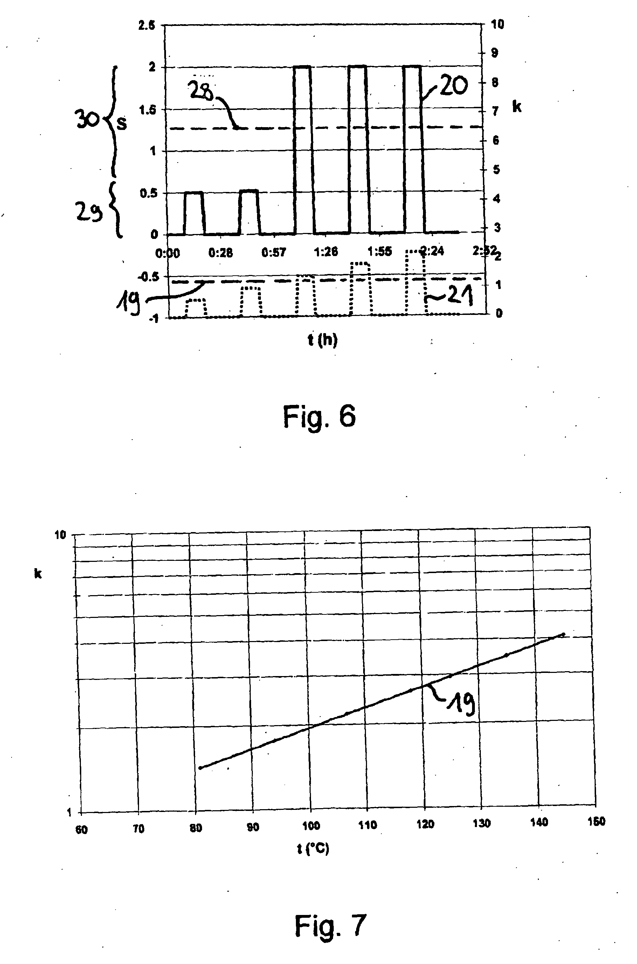

In Fig. 7 ist erkennbar, dass der Konzentrations-Schwellenwert 19 von der Temperatur der aus der gassensitiven Schicht 7 und der Beschichtung 18 gebildeten Schichtenfolge abhängig ist und mit zunehmender Temperatur kontinuierlich ansteigt. Dabei erfolgt der Anstieg etwa exponentiell mit der Temperatur. Gegebenenfalls kann der exponentielle Anstieg in dem für die Konzentrationsmessung relevanten Bereich linear angenähert werden.

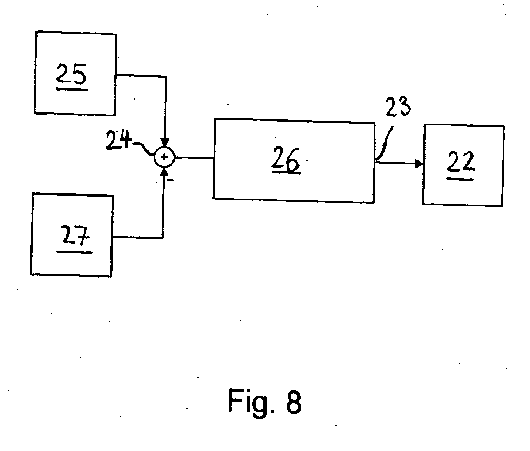

Die in Fig. 1-3 abgebildeten Gassensoren weisen jeweils eine in Fig. 8 nur schemafisch dargestellte Temperiereinrichtung 22 auf, mittels der die Temperatur der gassensitiven Schicht 7 und der Beschichtung 18 einstellbar ist. Ein Steuereingang der Temperiereinrichtung 22 ist mit einem Stellsignalausgang 23 einer Regeleinrichtung verbunden, die dazu dient, die Temperatur des der gassensitiven Schicht 7 und der Beschichtung 18 jeweils so einzustellen, dass das Sensorsignal 20 des Potentialsensors 27 im Wesentlichen von der Gaskonzentration unabhängig ist und in dem zweiten Aussteuerungsbereich 30 liegt.

Die Regeleinrichtung hat eine Vergleichseinrichtung 24, die einen mit dem Potentialsensor 27 verbunden Istwerteingang und einen mit einem Sollwertgeber 25 verbundenen Sollwerteingang aufweist. Ein Ausgang der Vergleichseinrichtung 24 ist über einen Regler 26 mit dem Stellsignalausgang 23 verbunden. Mit Hilfe des Sollwertgebers 25 wird ein Sollwert 28 an den Sollwerteingang angelegt, der im zweiten Aussteuerungsbereich 30 liegt, also einem Wert entspricht, den das Sensorsignal 20 des Potentialsensors 27 bei einer Wasserstoffgaskonzentration oberhalb des Konzentrations-Schwellenwerts 19 aufweist.

In einer ersten Betriebsart des Gassensors 1 steuert der Regler 26 die Temperiereinrichtung 22 jeweils derart an, dass beim Auftreten einer Abweichung zwischen dem Sensorsignal 20 des Potentialsensors 27 und dem Sollwert 28 die Temperatur der gassensitiven Schicht 7 und der Beschichtung 18 im Sinne einer Reduzierung der Abweichung verändert wird. Wenn das Sensorsignal 20 des Potentialsensors 27 mit dem Sollwert 28 übereinstimmt, ist die Temperatur der gassensitiven Schicht 7 und der Beschichtung 18 ein Maß für die Wasserstoffgaskonzentration.

In einer zweiten Betriebsart wird mit Hilfe der Temperiereinrichtung 22 die Temperatur der gassensitiven Schicht 7 und der Beschichtung 18 auf einen konstanten Wert eingestellt. Alternativ kann die Temperiereinrichtung 22 in der zweiten Betriebsart auch abgeschaltet sein, so dass die Temperatur des Gassensors 1 dann etwa der Umgebungstemperatur entspricht. Die zweite Betriebsart ist immer dann aktiviert, wenn die von dem Regler 26 ermittelte Temperatur einen vorgegebenen Temperatur-Mindestwert unterschreitet. Dieser kann beispielsweise etwa 60 - 80 °C betragen.

In der zweiten Betriebsart wird die Wasserstoffgaskonzentration in Abhängigkeit vom Signalwert des Sensorsignals 20 des Potentialsensors 27 und in Abhängigkeit von Kenngrößen ermittelt, die zum Beispiel in Form einer Kennlinie vorliegen können. In dem zweiten Betriebszustand entspricht die Signalauswertung im Wesentlichen der eines herkömmlichen Gassensors. Sobald der Temperatur-Mindestwert überschritten wird, wird auf die erste Betriebsart umgeschaltet, um die Wasserstoffgaskonzentration in Abhängigkeit von der eingestellten Temperatur zu bestimmen. Die erste Betriebsart kommt also bei hohen Wasserstoffgaskonzentrationen und die zweite Betriebsart bei geringeren Wasserstoffgaskonzentrationen zur Anwendung.

Die erste Betriebsart wird vorzugsweise gewählt, wenn die Konzentration des Wasserstoffgases zwischen 1 % und 4% beträgt. Der entsprechende Konzentrationsbereich kann experimentell ermittelt werden. In diesem Bereich ergibt sich ein annähernd exponentieller Zusammenhang zwischen der Temperatur und der Wasserstoffgaskonzentration. Der erfindungsgemäße Gassensor 1 ermöglicht dadurch in diesem Konzentrationsbereich im Vergleich zu einem herkömmlichen Gassensor eine deutlich verbesserte Auflösung.

## Patentansprüche

1. Gassensor (1) mit mindestens einer gassensitiven Schicht (7), die wenigstens einen Oberflächenbereich (9) aufweist, in dem die Austrittsarbeit von der Konzentration von mit dem Oberflächenbereich (9) in Kontakt bringbarem Wasserstoffgas abhängig ist, und mit zumindest einem über einen Luftspalt (8) kapazitiv an den Oberflächenbereich (9) gekoppelten elektrischen Potentialsensor (27), wobei der Oberflächenbereich (9) der gassensitiven Schicht (7) von einer für das Wasserstoffgas inerten, elektrisch isolierenden Beschichtung (18) überdeckt ist, die haftend mit der gassensitiven Schicht (7) verbunden und derart ausgestaltet ist, dass sie für das Wasserstoffgas und an dem Oberflächenbereich (9) adsorbierbarem Sauerstoffgas durchlässig ist, wobei die Beschichtung (1 8) für das Wasserstoffgas und das Sauerstoffgas unterschiedliche Diffusionskonstanten aufweist, und wobei die Diffusionskonstanten derart aufeinander abgestimmt sind, dass die Messempfindlichkeit des Gassensors (1) für das Wasserstoffgas zunimmt, wenn die Konzentration des Wasserstoffgases bei Anwesenheit des Sauerstoffgases einen vorbestimmten Konzentrations-Schwellenwert (19) überschreitet, **dadurch gekennzeichnet, dass** die Beschichtung (1 8) Polymethylmethacrylat enthält.

2. Gassensor (1) nach Anspruch 1, **dadurch gekennzeichnet, dass** die Dicke der Beschichtung (18) zwischen 0,3 µm und 4 µm und vorzugsweise zwischen 0,5 µm und 2,5 µm beträgt.

3. Gassensor (1) nach Anspruch 1 oder 2, **dadurch gekennzeichnet, dass** der Gassensor (1) zum Einstellen der Temperatur der gassensitiven Schicht (7) und der Beschichtung (18) eine Temperiereinrichtung, vorzugsweise eine Heizung, aufweist.

4. Gassensor (1) nach Anspruch 3, **dadurch gekennzeichnet, dass** er einen Sollwertgeber (25) und eine Regeleinrichtung aufweist, die einen mit dem Potentialsensor (27) verbundenen Istwerteingang, einen mit dem Sollwertgeber (25) verbundenen Sollwerteingang und einen mit der Temperiereinrichtung (22) verbundenen Stellsignalausgang (23) aufweist, und dass zum Anpassen des Konzentrations-Schwellenwerts (19) an die Wasserstoffgaskonzentration (21) mit Hilfe des Sollwertgebers (25) ein Sollwert (28) an den Sollwerteingang anlegbar ist, der einem Wert entspricht, den das Sensorssignal (20) des Potentialsensors (27) am Konzentrations-Schwellenwert (19) oder bei einer Wasserstoffgaskonzentration oberhalb des Konzentrations-Schwellenwerts (19) aufweist.

5. Gassensor (1) nach einem der Ansprüche 1 bis 4, **dadurch gekennzeichnet, dass** die gassensitive Schicht (7) aus Platin oder Palladium besteht.

6. Gassensor (1) nach einem der Ansprüche 1 bis 5, **dadurch gekennzeichnet, dass** der Potentialsensor (27) ein Feldeffekttransistor ist, der ein Substrat (2) aufweist, auf dem eine Drain (3) und eine Source (4) angeordnet sind, dass zwischen Drain (3) und Source (4) ein Kanalbereich (5) gebildet ist, und dass der Kanalbereich (5) direkt über den Luftspalt (8) oder indirekt über eine mit dem Kanalbereich (5) zusammenwirkende Gateelektrode (11) und eine mit der Gateelektrode (11) leitend verbundene Sensorelektrode (1 3) kapazitiv an den Oberflächenbereich (9) der gassensitiven Schicht (7) gekoppelt ist.

7. Gassensor (1) nach einem der Ansprüche 1 bis 6, **dadurch gekennzeichnet, dass** er als Kelvinsonde ausgestaltet ist, bei welcher der Potentialsensor (27) über eine durch den Luftspalt (8) von dem Oberflächenbereich (9) der gassensitiven Schicht (7) beabstandete, auf die gassensitive Schicht (7) zu- und von dieser wegbewegbaren Elektrode (15) kapazitiv an den Oberflächenbereich (9) der gassensitiven Schicht (7) gekoppelt ist.

## Claims

1. Gas sensor (1) with at least one gas-sensitive layer (7) having at least one surface region (9), in which the output work is dependent on the concentration of hydrogen gas able to be brought into contact with the surface region (9), and with at least one electrical potential sensor (27) capacitively coupled to the surface region (9) by way of an air gap (8), wherein the surface region (9) of the gas-sensitive layer (7) is covered by an electrically insulating coating (18), which is inert with respect to the hydrogen gas and is adhesively connected with the gas-sensitive layer (7) and which is so formed that it is permeable by hydrogen gas and oxygen gas which can be absorbed at the surface region (9), wherein the coating (18) has different diffusion constants for the hydrogen gas and the oxygen gas and wherein the diffusion constants are so matched to one another that the measuring sensitivity of the gas sensor (1) for the hydrogen gas increases when the concentration of the hydrogen gas exceeds a predetermined concentration threshold value (19) in the presence of the oxygen gas, **characterised in that** the coating (18) contains polymethylmethacrylate.

2. Gas sensor (1) according to claim 1, **characterised in that** the thickness of the coating (18) is between 0.3 microns and 4 microns and preferably between 0.5 microns and 2.5 microns.

3. Gas sensor (1) according to claim 1 or 2, **characterised in that** the gas sensor (1) has a temperature influencing device, preferably heating means, for setting the temperature of the gas-sensitive layer (7) and the coating (18).

4. Gas sensor (1) according to claim 3, **characterised in that** it comprises a target value transmitter (25) and a regulating device, which has an actual value input connected with the potential sensor (27), a target value input connected with the target value transmitter (25) and a setting signal output (23) connected with the temperature influencing device (22), and that for adaptation of the concentration threshold value (19) to the hydrogen gas concentration (21) a target value (28) can be applied with the help of the target value transmitter (25) to the target value input, which target value corresponds with a value which the sensor signal (20) of the potential sensor (27) has at the concentration threshold value (19) or when the hydrogen gas concentration is above the concentration threshold value (19).

5. Gas sensor (1) according to any one of claims 1 to 4, **characterised in that** the gas-sensitive layer (7) consists of platinum or palladium.

6. Gas sensor (1) according to any one of claims 1 to 5, **characterised in that** the potential sensor ((27) is a field effect transistor, which comprises a substrate (2) on which a drain (3) and a source (4) are arranged, that a channel region (5 is formed between the drain (3) and the source (4) and that the channel region (5) is capacitively coupled to the surface region (9) of the gas-sensitive layer (7) directly by way of the air gap (8) or indirectly by way of a gate electrode (11), which co-operates with the channel region (5), and a sensor electrode (13), which is conductively connected with the gate electrode (11).

7. Gas sensor (1) according to any one of claims 1 to 6, **characterised in that** it is constructed as a Kelvin probe, in which the potential sensor (27) is capacitively coupled to the surface region (9) of the gas-sensitive layer (7) by way of an electrode (15) spaced by the air gap (8) from the surface region (9) of the gas-sensitive layer (7) and movable towards and away from the gas-sensitive layer (7).

## Revendications

1. Capteur de gaz (1) avec au moins une couche sensible au gaz (7), qui présente au moins une zone de surface (9) dans laquelle le travail de sortie dépend de la concentration de gaz hydrogène pouvant être mis en contact avec la zone de surface (9), et avec au moins un capteur de potentiel électrique (27) couplé de façon capacitive à la zone de surface (9) au moyen d'un entrefer (8), dans lequel la zone de surface (9) de la couche sensible au gaz (7) est recouverte d'un revêtement (18) électriquement isolant, inerte pour le gaz hydrogène, qui est lié par adhérence à la couche sensible au gaz (7) et qui est conçu de telle manière qu'il soit perméable au gaz hydrogène et au gaz oxygène adsorbable sur la zone de surface (9), dans lequel le revêtement (18) présente des constantes de diffusion différentes pour le gaz hydrogène et le gaz oxygène et dans lequel les constantes de diffusion sont accordées l'une à l'autre de telle manière que la sensibilité de mesure du capteur de gaz (1) pour le gaz hydrogène augmente lorsque la concentration du gaz hydrogène dépasse une valeur de seuil de concentration prédéterminée (19) en présence du gaz oxygène, **caractérisé en ce que** le revêtement (18) contient du polyméthacrylate de méthyle.

2. Capteur de gaz (1) selon la revendication 1, **caractérisé en ce que** l'épaisseur du revêtement (18) est comprise entre 0,3 µm et 4 µm, et de préférence entre 0,5 µm et 2,5 µm.

3. Capteur de gaz (1) selon la revendication 1 ou 2, **caractérisé en ce que** le capteur de gaz (1) présente un dispositif de réglage de la température, de préférence un chauffage, pour le réglage de la température de la couche sensible au gaz (7) et du revêtement (18).

4. Capteur de gaz (1) selon la revendication 3, **caractérisé en ce qu'**il présente un générateur de valeur de consigne (25) et un dispositif de régulation, qui présente une entrée de valeur réelle reliée au capteur de potentiel (27), une entrée de valeur de consigne reliée au générateur de valeur de consigne (25) et une sortie de signal de réglage (23) reliée au dispositif de réglage de la température (22), et **en ce que**, pour l'adaptation de la valeur de seuil de la concentration (19) à la concentration du gaz hydrogène (21) à l'aide du générateur de valeur de consigne (25), on peut appliquer à l'entrée de valeur de consigne une valeur de consigne (28), qui correspond à une valeur que le signal de capteur (20) du capteur de potentiel (27) présente à la valeur de seuil de la concentration (19) ou pour une concentration de gaz hydrogène supérieure à la valeur de seuil de la concentration (19).

5. Capteur de gaz (1) selon l'une quelconque des revendications 1 à 4, **caractérisé en ce que** la couche sensible au gaz (7) se compose de platine ou de palladium.

6. Capteur de gaz (1) selon l'une quelconque des revendications 1 à 5, **caractérisé en ce que** le capteur de potentiel (27) est un transistor à effet de champ, qui présente un substrat (2) sur lequel sont disposés un drain (3) et une source (4), **en ce qu'**une zone de canal (5) est formée entre le drain (3) et la source (4) et **en ce que** la zone de canal (5) est couplée de façon capacitive à la zone de surface (9) de la couche sensible au gaz (7) directement par l'entrefer (8) ou indirectement par une électrode de grille (11) coopérant avec la zone de canal (5) et une électrode de capteur (13) reliée de façon conductrice à l'électrode de grille (11).

7. Capteur de gaz (1) selon l'une quelconque des revendications 1 à 6, **caractérisé en ce qu'**il est conçu comme une sonde de Kelvin, dans laquelle le capteur de potentiel (27) est couplé de façon capacitive à la zone de surface (9) de la couche sensible au gaz (7) par une électrode (15) espacée de la zone de surface (9) de la couche sensible au gaz (7) par l'entrefer (8) et pouvant être rapprochée de la couche sensible au gaz (7) et écartée de celle-ci.
